(19) Europäisches Patentamt
European Patent Office
Office européen des brevets

(11) **EP 3 291 923 B1**

(12) **FASCICULE DE BREVET EUROPEEN**

(45) Date de publication et mention
de la délivrance du brevet:
**01.03.2023 Bulletin 2023/09**

(21) Numéro de dépôt: **16725055.4**

(22) Date de dépôt: **09.05.2016**

(51) Classification Internationale des Brevets (IPC):
**B06B 1/02** *(2006.01)*   **A61N 7/00** *(2006.01)*

(52) Classification Coopérative des Brevets (CPC):
**B06B 1/02; A61N 7/02;** A61B 2017/00084;
A61B 2017/00725

(86) Numéro de dépôt international:
**PCT/EP2016/060347**

(87) Numéro de publication internationale:
**WO 2016/177910 (10.11.2016 Gazette 2016/45)**

(54) **PROCEDE D'AJUSTEMENT DE PARAMETRES DE FONCTIONNEMENT POUR L'ALIMENTATION D'UN TRANDUCTEUR**

VERFAHREN ZUM EINSTELLEN VON BETRIEBSPARAMETERN ZUR STROMVERSORGUNG EINES WANDLERS

METHOD FOR ADJUSTING OPERATING PARAMETERS FOR THE POWER SUPPLY OF A TRANSDUCER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2015 FR 1554143**

(43) Date de publication de la demande:
**14.03.2018 Bulletin 2018/11**

(73) Titulaire: **Eye Tech Care**
**69140 Rillieux-la-Pape (FR)**

(72) Inventeurs:
• **CHARREL, Thomas**
**01390 Mionnay (FR)**

• **BAFFIE, Laure**
**01120 Thil (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**WO-A1-2011/148314    US-A1- 2004 082 857**
**US-A1- 2014 024 975**

**Description**

**DOMAINE DE L'INVENTION**

La présente invention concerne le domaine technique général des dispositifs et procédés de traitement d'une pathologie, telle qu'une pathologie oculaire, en utilisant la technique dite des ultrasons focalisés de haute intensité (ou *« HIFU »*, acronyme de l'expression anglaise *« High-Intensity Focused Ultrasound »*).

**ARRIERE PLAN DE L'INVENTION**

On connaît différents procédés et dispositifs basés sur la technologie des HIFU pour traiter une pathologie.

Le document WO 2009/103721 décrit notamment un dispositif de traitement d'une pathologie oculaire incluant une sonde composée d'un anneau et de moyens de génération d'ultrasons.

L'anneau présente une partie proximale destinée à être en contact avec un oeil d'un patient, et une partie distale destinée à recevoir les moyens de génération d'ultrasons.

Les moyens de génération d'ultrasons présentent un profil concave. Plus précisément, les moyens de génération d'ultrasons comprennent six transducteurs en forme de segment de cylindre positionnés sur une couronne cylindrique d'axe A-A'. Les moyens de génération d'ultrasons comprennent également un câble électrique destiné à être connecté à une source d'alimentation électrique pour permettre l'alimentation en énergie des transducteurs.

Le principe d'utilisation d'un tel dispositif est le suivant. Une fois la sonde mise en place sur l'organe à traiter, la source d'alimentation électrique est activée. Les transducteurs entrent en vibration et génèrent des ultrasons qui focalisent sur une zone cible, induisant son augmentation de température, provoquant notamment la coagulation des tissus chauffés au niveau de cette zone cible.

A chaque traitement, une sonde usagée doit être remplacée par une sonde neuve.

Or, les performances des sondes peuvent varier d'une sonde à l'autre, notamment du fait de jeux mécaniques lors de l'assemblage des transducteurs et/ou des tolérances de fabrication (variations de la rugosité de surface ou de l'épaisseur de chaque transducteur, etc.).

Ainsi, la température atteinte dans la zone cible peut varier entre deux sondes successives (et même entre deux transducteurs d'une même sonde).

C'est pourquoi il est nécessaire de calibrer chaque transducteur d'une sonde pour déterminer l'énergie à lui appliquer en fonction de la température souhaitée dans la zone cible à traiter.

Cette température souhaitée peut varier en fonction du type de traitement que l'on désire réaliser. Par exemple, pour certains traitements d'un organe, il peut être préférable d'induire une augmentation faible de la température pendant un temps important de sorte à provoquer une *« coagulation lente »* de la zone cible ; alors que pour d'autres traitements, une augmentation importante de la température pendant un temps court sera privilégié de sorte à provoquer une *« coagulation rapide »* de la zone cible.

Cette température souhaitée peut également varier en fonction de la zone cible ou de l'organe à traiter. Par exemple, la température souhaitée sera différente dans le cas du traitement de corps ciliaire par rapport au traitement de cellules cancéreuses du foie.

Il est donc nécessaire :

- d'une part de définir des paramètres de calibration des sondes en fonction de l'application visée, et
- d'autre part de calibrer chaque transducteur d'une sonde préalablement à son utilisation.

Le document US 2014/024975 décrit un dispositif et un procédé pour augmenter la précision de la puissance ultrasonore délivrée à un patient par un système HIFU ayant des composants échangeables. Les composants du système comprennent un ou plusieurs dispositifs de mémoire dans lesquels les paramètres d'étalonnage du composant sont stockés, par exemple lorsque le composant est fabriqué. Pendant le fonctionnement du système, les données des paramètres d'étalonnage sont reçues de la mémoire et utilisées par le système pour ajuster la puissance de sortie d'une ou plusieurs sources d'ultrasons et / ou des niveaux de signal reçus en fonction des paramètres d'étalonnage reçus. Les paramètres d'étalonnage peuvent comprendre des données relatives à l'impédance, la sensibilité, les fonctions de transfert, la linéarité ou d'autres données qui pourraient affecter la puissance délivrée à un patient par le composant.

Un but de la présente invention est de proposer une solution pour l'ajustement de paramètres d'alimentation d'une sonde de traitement d'une pathologie basée sur la technique des HIFU.

**BREVE DESCRIPTION DE L'INVENTION**

A cet effet, l'invention propose un procédé de calibration d'une sonde de traitement comportant au moins un transducteur destiné à générer des ultrasons focalisés de haute intensité dans une zone cible de focalisation, la sonde étant destinée à être connectée électriquement à une source d'alimentation pour la fourniture d'un signal électrique d'alimentation du transducteur, remarquable en ce que le procédé comprend une étape d'ajustement d'une fréquence du signal électrique d'alimentation du transducteur de sorte que la variation entre l'intensité maximale de vibration et l'intensité minimale de vibration du transducteur le long de sa largeur soit minimale.

Selon l'invention, l'étape d'ajustement de la fréquence d'excitation du transducteur comprend les sous-étapes consistant à :

○ alimenter le transducteur avec une pluralité de signaux d'entrée ayant des amplitudes égales et des fréquences respectives différentes,
○ mesurer des températures (/pressions) en différents points de la zone cible pour chaque signal d'entrée
○ obtenir un profil de température (/pression) du transducteur pour chaque signal d'entrée,
○ sélectionner parmi la pluralité de profils de température (/pression) du transducteur, le profil de température (/pression) pour lequel la variation entre la température (/pression) maximale et minimale du profil de température (/pression) est la plus faible le long de la largeur du transducteur,
○ calculer la fréquence du signal électrique d'alimentation du transducteur à partir de la fréquence du signal d'entrée associé au profil de température (/pression) sélectionné ;

- le procédé peut comprendre en outre une étape de détermination d'une fréquence de résonnance du transducteur ;
- les fréquences pluralité de signaux d'entrée peuvent être comprises entre 90% et 110% de la fréquence de résonnance du transducteur ;
- le procédé peut comprendre en outre une étape d'ajustement d'une tension du signal électrique d'alimentation du transducteur en fonction d'une température de consigne dans la zone cible de focalisation ;
- l'étape d'ajustement d'une tension d'excitation peut comprendre les sous étapes consistant à :

a) alimenter le transducteur avec un signal d'entrée de fréquence fixe, notamment égale à la fréquence d'excitation,
b) mesurer des températures en différents points de la zone cible de focalisation,
c) calculer une température moyenne à partir des températures mesurées,
d) comparer la température moyenne calculée à la température de consigne,
e) si la température moyenne est supérieure à la température de consigne, alors diminuer l'amplitude du signal d'entrée et répéter les opérations b) à e)
f) si la température moyenne est inférieure à la température de consigne, alors augmenter l'amplitude du signal d'entrée et répéter les opérations b) à f),
g) si la température moyenne est égale à la température de consigne, choisir comme tension d'excitation la valeur de la tension du signal d'entrée.

L'invention concerne également une sonde de traitement d'une pathologie comportant des moyens de générations d'ultrasons incluant au moins un transducteur pour générer des ultrasons focalisés de haute intensité dans une zone cible de focalisation selon la revendication 6.

Avantageusement, les moyens d'ajustement peuvent comprendre une carte électronique de pilotage de la source d'alimentation, ladite carte incluant une mémoire contenant au moins un paramètre de calibration obtenu en mettant en oeuvre le procédé de calibration décrit ci-dessus.

De préférence, la carte électronique de pilotage est intégrée dans les moyens de génération d'ultrasons.

## BREVE DESCRIPTION DES DESSINS

D'autres avantages et caractéristiques du procédé selon l'invention et du produit associé ressortiront mieux de la description qui va suivre de plusieurs variantes d'exécution, données à titre d'exemples non limitatifs, à partir des dessins annexés sur lesquels :

- la figure 1 illustre schématiquement un dispositif de traitement
- la figure 2 illustre schématiquement un procédé d'ajustement de paramètres de fonctionnement d'une sonde de traitement,
- la figure 3 illustre un profil de vibration d'un transducteur,
- la figure 4 illustre une suite de profils de température obtenus pour des signaux d'entrée de fréquence variable
- la figure 5 est un abaque de courbes représentatives d'une température dans un gel en fonction d'une durée d'exposition à des ultrasons.

## DESCRIPTION DETAILLEE DE L'INVENTION

On va maintenant décrire plus en détails des exemples de dispositif et de procédé selon l'invention en référence aux figures. Dans ces différentes figures, les éléments équivalents sont désignés par la même référence numérique.

### 1. Dispositif de traitement d'une pathologie

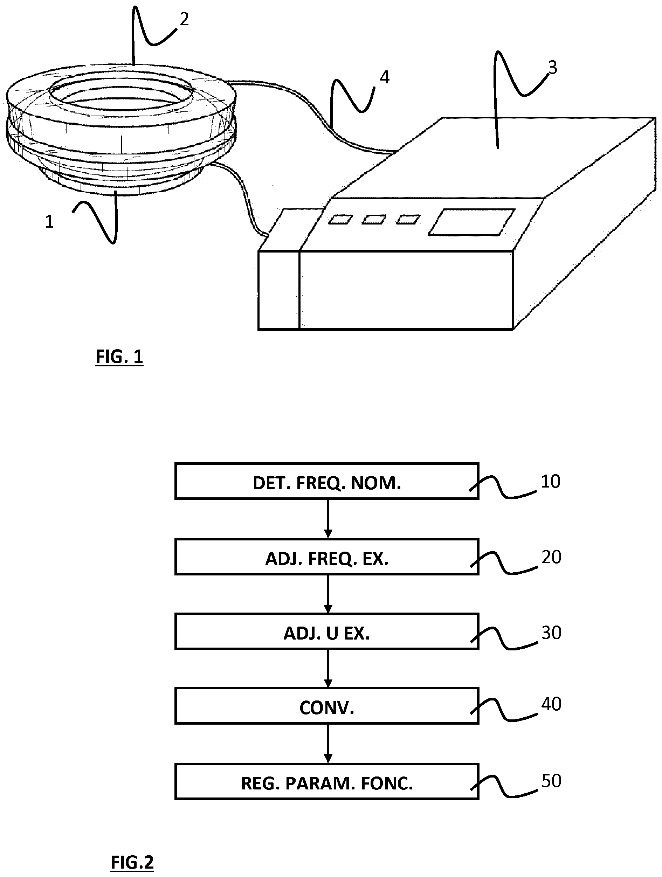

On a illustré à la figure 1 un exemple de dispositif basé sur la technologie des HIFU pour le traitement d'une pathologie oculaire.

Le dispositif comprend un support 1 pour des moyens de génération d'ultrasons 2 connectés électriquement à une source d'alimentation électrique 3 par l'intermédiaire d'un câble électriquement conducteur de tout type connu par l'homme du métier.

Le support est en forme d'un anneau qui est particulièrement adapté pour le traitement d'une pathologie oculaire. Bien entendu, ce dispositif pourrait être adapté pour permettre le traitement d'autres organes que l'œil, notamment en modifiant la forme du support 1 et/ou l'agencement des moyens de génération d'ultrason 2.

La source d'alimentation électrique 3 est adaptée pour délivrer des oscillations électriques aux moyens de génération d'ultrasons. Ces oscillations électriques peuvent être délivrées en mode continu (i.e. oscillations électriques en permanence) ou en mode cyclique (i.e. succession de périodes, une période comportant un premier intervalle de temps incluant une (ou plusieurs) oscillation(s) électrique(s) et un deuxième intervalle de temps ne comprenant pas d'oscillation électrique). En d'autres termes, le générateur est adapté pour générer un (ou plusieurs) train(s) d'impulsions électriques. De préférence, la source d'alimentation électrique 3 est utilisée en mode continu pour augmenter l'effet d'échauffement des tissus ciblés (comparé à une source d'alimentation utilisée en mode cyclique par exemple).

Les moyens de génération d'ultrasons 2 permettent de générer une énergie ultrasonore. Ils sont disposés de manière adaptée à la pathologie à traiter. Pour le traitement d'une pathologie oculaire, ils comprennent une couronne annulaire comportant :

- des première et deuxième bases opposées,
- une paroi latérale externe entre les première et deuxième bases, et
- une paroi latérale interne définissant un canal central entre les première et deuxième bases.

La première base comporte au moins un transducteur ayant un élément rayonnant pour la génération d'ultrasons.

Le profil du (ou des) élément(s) rayonnant(s) peut être adapté pour permettre l'orientation et la focalisation des ultrasons sur une zone cible donnée correspondant à un segment de droite, comme il sera décrit plus en détails dans la suite.

En variante, le transducteur peut comprendre un (ou des) réflecteur(s) pour réfléchir, orienter et focaliser en une zone cible donnée les ultrasons générés par l'élément (ou les éléments) rayonnant(s).

Dans un mode de réalisation, six transducteurs s'étendent sur la première base de la couronne. Les transducteurs sont regroupés en deux paires de trois transducteurs séparés par deux secteurs inactifs. Les secteurs inactifs sont localisés sur la couronne de sorte à s'étendre dans un plan temporal/nasal du patient lorsque la sonde est mise en place sur l'œil, ces secteurs correspondant à des zones de l'œil incluant la majorité des terminaisons nerveuses et vascularités.

Les moyens de génération d'ultrasons peuvent également comprendre une carte électronique de pilotage de la source de puissance alimentant les moyens de génération d'ultrasons.

Cette carte comprend une mémoire dans laquelle sont stockés des paramètres de fonctionnement pour chaque transducteur. Ces paramètres de fonctionnement permettent de piloter la source d'alimentation en fonction des caractéristiques techniques associées à chaque transducteur.

En effet, du fait des tolérances de fabrication des sondes (tolérances dimensionnelles, géométriques, d'usinage, etc.), deux transducteurs alimentés à des tension et fréquence électriques identiques peuvent avoir des réponses acoustiques différentes.

Notamment, la fréquence de résonance de chaque transducteur peut varier de quelques dizaines de pourcent d'un transducteur à l'autre. De même, le rendement et/ou la réponse acoustique de chaque transducteur peut varier d'un transducteur à l'autre. Ainsi, il peut être nécessaire d'ajuster la tension et la fréquence appliquées à chaque transducteur d'une sonde de sorte à homogénéiser leurs effets au niveau de leurs zones cibles respectives.

La carte électronique permet de piloter :

- un courant électrique de fonctionnement,
- une tension électrique de fonctionnement et/ou
- une fréquence de fonctionnement

appliqués à chaque transducteur d'une sonde par la source d'alimentation de sorte à homogénéiser les réponses thermiques desdits transducteurs aux niveaux de leurs zones cibles respectives (i.e. zones de focalisation des ultrasons).

Dans certains modes de réalisation, la carte électronique est implantée dans la source d'alimentation 3. Dans ce cas, les paramètres de fonctionnement des transducteurs d'une sonde doivent être renseignés dans la source d'alimentation 3 pour chaque changement de sonde.

Dans d'autres modes de réalisation, la carte électronique est intégrée dans chaque sonde, par exemple logée dans la couronne. Ceci permet de disposer d'une sonde totalement calibrée sans nécessiter la mise en oeuvre d'une quelconque opération de réglage de la source d'alimentation 3 par l'utilisateur. On limite ainsi les risques d'erreur et on simplifie l'utilisation du dispositif de traitement.

## 2. *Procédé d'ajustement des paramètres de fonctionnement d'une sonde*

On va maintenant décrire un exemple de procédé d'ajustement des paramètres de fonctionnement d'une sonde.

Ce procédé permet, pour chaque transducteur d'une sonde, de déterminer les paramètres de fonctionnement enregistrés dans la mémoire de la carte électronique pour le traitement d'un tissu.

Le procédé d'ajustement comprend des étapes réalisées sur un banc de test pour déterminer une fréquence optimal d'excitation et une tension optimale d'excitation pour chaque transducteur.

### 2.1. *Détermination de la fréquence nominale de chaque transducteur*

En référence à la figure 2, le procédé d'ajustement comprend une étape 10 consistant à déterminer la fréquence nominale (ou fréquence de résonance) de chaque transducteur d'une sonde.

Chaque transducteur est composé d'un matériau piézo-électrique apte à vibrer lorsqu'il est soumis à une tension électrique de fréquence donnée. Ces vibrations sont alors susceptibles, lorsque la fréquence de résonance du transducteur est atteinte (vibration maximale) de se transmettre au milieu environnant sous forme d'ondes ultrasonores.

La fréquence nominale d'un transducteur peut par exemple être déterminée selon la méthode suivante. On connecte le transducteur à un analyseur de réseau qui permet de mesurer une impédance électrique sur une plage de fréquence donnée.

La fréquence nominale correspond alors à la fréquence électrique pour laquelle l'adaptation d'impédance est optimale (i.e. fréquence électrique pour laquelle la vibration du transducteur est maximale) ce qui se traduit au niveau électrique par un rapport entre un signal électrique reçu divisé par un signal électrique émis minimal.

Pour une sonde comportant six transducteurs, l'étape de détermination sera mise en oeuvre six fois afin de déterminer la fréquence nominale de chaque transducteur respectif.

### 2.2. *Ajustement de la fréquence d'excitation*

Le procédé comprend de plus une étape 20 d'ajustement de la fréquence d'excitation de chaque transducteur autour de sa fréquence nominale.

L'ajustement de la fréquence d'excitation d'un transducteur permet une répartition plus homogène de l'énergie acoustique qu'il produit sur toute sa largeur L (correspondant à la génératrice de la portion de cylindre constituant le transducteur).

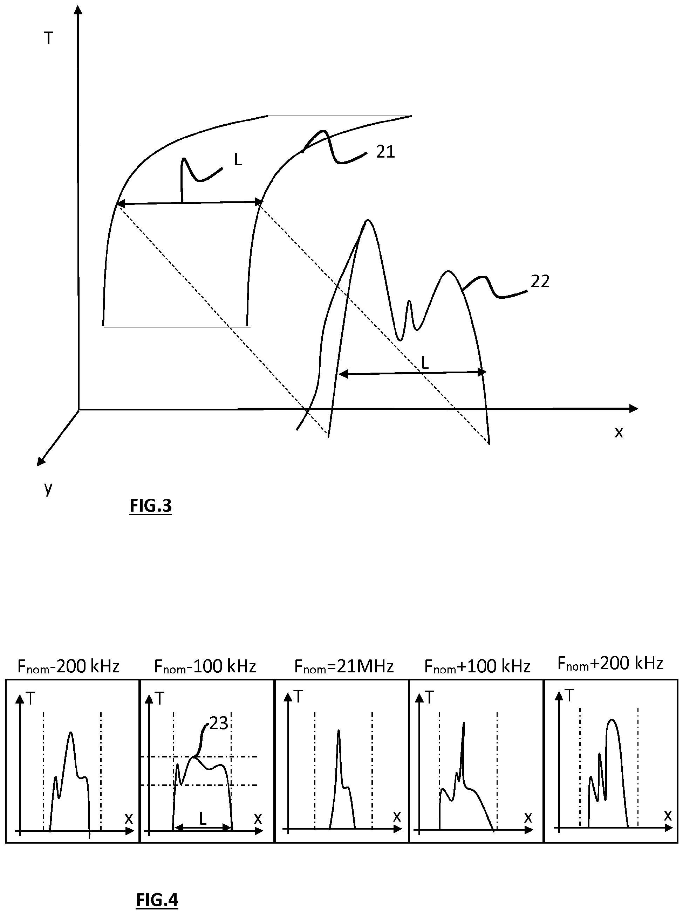

En effet et comme le montre le profil de vibration 22 illustré à la figure 3, un transducteur 21 ne vibre pas de façon uniforme sur toute sa largeur L de sorte que les tailles de lésions générées par deux transducteurs de mêmes dimensions peuvent varier.

Pour un transducteur donné, l'ajustement de la fréquence d'excitation consiste à :

- connecter le transducteur à un générateur (de tension) pour appliquer un signal d'entrée (i.e. tension sinusoïdale) au transducteur,
- positionner un dispositif de mesure de température - tel qu'un thermocouple - dans la zone cible du transducteur.

On fait varier la fréquence du signal d'entrée dans une gamme de valeurs autour de la fréquence nominale du transducteur ($\pm 10\%$ de la fréquence nominale), par exemple dans une gamme de fréquences comprise entre des fréquences minimale et maximale telles que :

- la fréquence minimale soit égale à 90% de la fréquence nominale,
- la fréquence maximale soit égale à 110% de la fréquence nominale.

Le dispositif de mesure permet d'obtenir un profil de température pour chaque signal d'entrée de fréquence différente. Ce profil de température est représentatif du profil de vibration du transducteur.

On sélectionne ensuite comme fréquence d'excitation, la fréquence du signal d'entrée pour laquelle la variation entre la température maximale et la température minimale du profil de température est la plus faible.

Par exemple, en référence à la figure 4 illustrant une variation du profil de température d'un transducteur pour un signal d'entrée dont la fréquence varie de $\pm 200$ kHz autour de la fréquence nominale (21 MHz) du transducteur, on sélectionnera comme fréquence d'excitation la fréquence nominale moins 100 kHz. En effet pour cette fréquence d'excitation, le profil de température 23 est le plus homogène sur toute la largeur du transducteur (et donc de la zone cible).

L'étape d'ajustement de la fréquence d'excitation d'un transducteur permet d'obtenir le profil de température de zone cible le plus homogène.

Lors de cette étape d'ajustement de la fréquence d'excitation, la tension du signal d'entrée est maintenue constante.

Une autre étape du procédé va consister à ajuster la tension d'excitation de chaque transducteur pour atteindre une température de consigne au niveau de la zone cible.

Pour une sonde comportant six transducteurs, l'étape d'ajustement sera mise en oeuvre six fois afin de déterminer une fréquence d'excitation pour chaque transducteur respectif.

### 2.3. *Ajustement de la tension d'excitation*

Une autre étape 30 du procédé consiste à ajuster la tension d'excitation appliquée à chaque transducteur par la source d'alimentation.

Comme indiqué précédemment, les rendements des transducteurs peuvent varier d'un transducteur à l'autre. Ainsi, la tension d'excitation doit être ajustée pour chaque transducteur afin que les transducteurs d'une sonde délivrent des quantités d'énergie acoustiques identiques au niveau de leurs zones cibles respectives.

En effet, même si l'étape d'ajustement de la fréquence d'excitation permet d'uniformiser le profil de température (et donc de vibration) de chaque transducteur d'une sonde, les profils des différents transducteurs d'une sonde présentent toutefois des différences entre eux. Or, la température dans la zone cible d'un transducteur dépend de son profil de vibration.

Il est donc nécessaire d'ajuster la tension d'excitation de chaque transducteur pour que :

- la température atteinte au niveau de sa zone cible corresponde à une température de consigne, et pour que
- les températures atteintes dans les zones cibles respectives des différents transducteurs d'une sonde soient sensiblement identiques.

Pour un transducteur donné, l'ajustement de la tension d'excitation consiste à connecter le transducteur à un générateur (de tension) pour appliquer un signal d'entrée (i.e. tension sinusoïdale) au transducteur, et à positionner un dispositif de mesure de température - tel qu'un thermocouple - dans la zone cible du transducteur.

On fait ensuite varier la tension du signal d'entrée jusqu'à ce que la température au niveau de la zone cible corresponde à la température de consigne.

Dans certaines variantes de réalisation, on considère une température moyenne sur la zone cible pour ajuster la tension d'excitation d'un transducteur.

Plus précisément, pour un signal d'entrée de tension donnée :

- on mesure les températures en différents points de la zone cible ;
- on calcule une température moyenne à partir des températures mesurées ;
- on compare cette température moyenne à une température de consigne ;
- on diminue (respectivement augmente) la tension du signal d'entrée si la température moyenne est supérieure (respectivement inférieure) à la température de consigne ;
- on assigne à la tension d'excitation la valeur de la tension du signal d'entrée si la température moyenne est égale à la température de consigne.

Dans d'autres variantes, on considère la température moyenne à mi-hauteur sur la zone cible. Ceci permet de limiter l'influence des pics de température dans l'ajustement de la tension d'excitation.

Pour une sonde comportant six transducteurs, l'étape d'ajustement sera mise en oeuvre six fois afin de déterminer une tension d'excitation pour chaque transducteur respectif.

2.4. *Enregistrement des paramètres de fonctionnement*

A l'issue des étapes de détermination et d'ajustement, on obtient pour chaque transducteur d'une sonde : une fréquence d'excitation et une tension d'excitation.

Celles-ci sont obtenues en utilisant un banc de test comportant :

- un support pour le positionnement des moyens de génération d'ultrason,
- une embase destinée à recevoir un bloc de gel thermosensible utilisé pour simuler le tissu destiné à être traité,
- un thermocouple destiné à être introduit dans le bloc de gel, au niveau de la zone cible de chaque transducteur pour mesurer des températures en différents points de la zone cible.

De préférence, le bloc de gel est un gel à base de protéine, plus précisément à base de protéine animale, et encore plus précisément à base d'albumine de sérum bovin (ou *« BSA »* selon le sigle anglais *« Bovine Serum Albumine »*). Un tel gel est notamment décrit dans la publication intitulée « Gel phantom for use in high-intensity focused ultrasound dosimetry » de Lafon C, Zderic V, Noble ML, Yuen JC, Kaczkowski PJ, Sapozhnikov OA, Chavrier F, Crum LA, Vaezy S paru dans Ultrasound Med Biol. 2005 Oct;31, Vol 31 (No 10): pages 1383-1389.

Un exemple de composition du gel est donné à titre indicatif dans le tableau suivant.

| COMPOSITION | PROPORTION |
|---|---|
| Eau dégazée (mL) | 143.08 |
| Acrylamide/Bis-Acrylamide 40% (mL) | 34.92 |
| TRIS 1 mol/L (mL) | 20 |
| BSA (g) | 36 |
| 10% APS (mL) | 1.68 |
| TEMED (mL) | 0.2 |

Avec :

- TRIS le trishydroxyméthylaminométhane, où 1L TRIS 1mol/L = 88,8g TRIZMA Hydrochloride + 53g de TRIS de base + 1L d'eau,
- TEMED le Tétraméthyléthylènediamine, et
- APS le PerSulfate d'ammonium, où 10mL de 10% APS = 1g APS + 10mL d'eau

Les caractéristiques de ce gel thermosensible présentent l'avantage d'être représentatives des caractéristiques d'un tissu.

Néanmoins, la tension d'excitation estimée sur le banc de test (pour la température de consigne) doit être transposée (étape 40) pour une utilisation de la sonde avec la source d'alimentation (celle-ci ayant une impédance caractéristique différente de celle du générateur utilisé sur le banc de test).

A cet effet, on applique une fonction de transfert à la tension d'excitation.

Les paramètres de fonctionnement (fréquence/tension transposée) associés à chaque transducteur sont enregistrés (étape 50) dans la mémoire de la carte électronique pour permettre le pilotage de la source d'alimentation 3 afin d'obtenir :

- pour chaque transducteur, un chauffage sensiblement homogène sur toute la surface de sa zone cible tissulaire,
- pour les différents transducteurs, des chauffages sensiblement homogènes des différentes zones cibles tissulaires.

3. *Procédé de détermination d'un (ou plusieurs) paramètre(s) de calibration d'une sonde de traitement en fonction d'une application visée*

On a vu précédemment que le procédé d'ajustement permet d'uniformiser les profils des vibrations générées par les transducteurs d'une sonde dans leurs zones cibles respectives.

Si la sonde est orientée vers un tissu, ces vibrations permettent de chauffer le tissu au niveau des zones cibles tissulaires.

Or, la température nécessaire (ci-après dénommée *« température souhaitée »*) dans la zone cible tissulaire pour traiter efficacement un tissu peut varier d'un tissu à l'autre.

Il en va de même de la durée nécessaire pour traiter efficacement un tissu qui peut varier en fonction du tissu à traiter ou du traitement à réaliser (ci-après dénommée *« durée souhaitée »*).

Ainsi, l'efficacité d'un traitement dépend d'un couple température souhaitée / durée souhaitée. Du choix de ce couple dépend la température de consigne utilisée dans le procédé d'ajustement décrit au point 2.

Les inventeurs ont constaté une corrélation entre la cinétique d'élévation de température observée dans un gel - à base d'albumine de sérum bovin, et notamment pour le gel donné dans le tableau ci-dessus - au niveau de la zone cible d'un transducteur en fonction de l'intensité acoustique dudit transducteur, cette intensité acoustique dépendant elle-même de la température de consigne utilisée pour calibrer le transducteur.

Pour permettre de déterminer rapidement une température de consigne en fonction d'une température souhaitée et/ou d'une durée souhaitée pour un traitement, les inventeurs ont estimé différentes courbes en utilisant un banc de test. Plus précisément, les inventeurs ont choisi un transducteur en tant qu'étalon de référence en fonction de certains critères de qualité (qualités mécaniques du transducteur et/ou la qualité de la lésion générée par le transducteur etc.).

Les inventeurs ont ensuite :

- calibré l'étalon de référence en tension pour différentes températures de consigne ; puis,
- pour chaque température de consigne, mesuré la variation de la température dans la zone cible en fonction du temps.

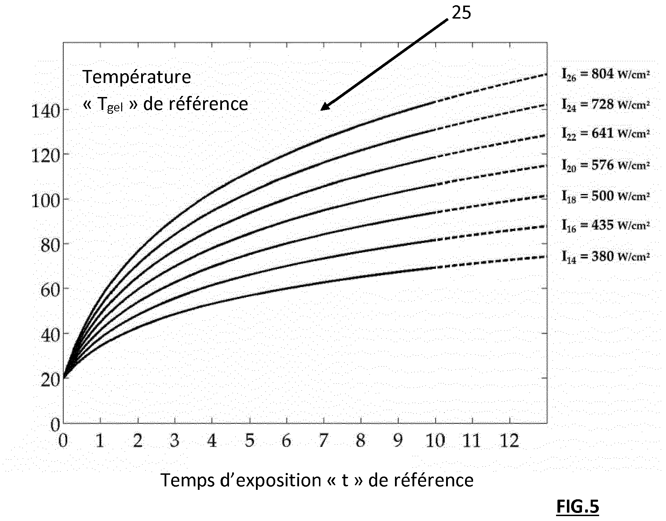

Les inventeurs ont ainsi obtenu une pluralité de courbes illustrées sur un abaque représenté à la figure 5, chaque courbe étant associée à une température de consigne respective.

Par exemple, les inventeurs ont déterminé une première tension d'excitation à appliquer au transducteur constituant l'étalon de référence pour que la température dans la zone cible augmente d'une température prédéterminée (par exemple 14°C) au bout d'une durée prédéterminée (par exemple une demi-seconde). Puis les inventeurs ont mesuré la variation de la température dans la zone cible en fonction du temps sur une période de temps (par exemple comprise entre 0 seconde et 30 minutes). Ils ont ainsi obtenu la courbe référencée $I_{14}$ sur l'abaque de la figure 5.

Les inventeurs ont ensuite déterminé une deuxième tension d'excitation à appliquer au transducteur constituant l'étalon de référence pour que la température dans la zone cible augmente de 16°C au bout d'une demi-seconde. Une fois le transducteur étalon calibré, les inventeurs ont mesuré la variation de la température dans la zone cible en fonction du temps sur une période de temps comprise entre 0 seconde et 30 minutes. Ils ont ainsi obtenu la courbe référencée $I_{16}$ sur l'abaque de la figure 5. Et ainsi de suite.

Ceci a permis d'aboutir à l'abaque représenté à la figure 5. Cet abaque est constitué de courbes illustrant chacune une température dans un gel en fonction d'une durée d'exposition aux ultrasons, lesdites courbes étant chacune associée à une température de consigne respective (utilisable dans le procédé d'ajustement décrit au point 2). En effet, la figure 5 illustre l'évolution d'une différence de température dans un gel thermosensible en fonction du temps pour des températures de consigne comprises entre 14°C et 26°C.

Comme indiqué ci-dessus, les caractéristiques de ce gel thermosensible présentent l'avantage d'être représentatives des caractéristiques d'un tissu, de sorte qu'il existe une corrélation entre :

- la température souhaitée dans un tissu et la température dans le gel,
- la durée d'exposition souhaitée dans un tissu et la durée d'exposition dans le gel.

Ainsi, ces courbes permettent indirectement une mise en relation d'une température de consigne en fonction d'un temps souhaité et d'une durée d'exposition souhaitée pour obtenir un traitement efficace dans un tissu.

La loi thermique qui régit les courbes illustrées à la figure 5 peut-être interpolée par une loi logarithmique de type :

$$T_i(t,K) = \log(t+1) \times K_i + Tamb \; ;$$

Avec :

- « t » une durée de traitement (ou temps d'insonification) variant de 0 à +inf,
- « Tamb » la température ambiante (ici 20°C) et
- « $K_i$ » un coefficient de pondération lié à l'intensité acoustique.

Le tableau suivant donne les valeurs obtenues pour le gel décrit dans le tableau précédent :

| I (ou Température de consigne) | Iac (intensité acoustique en W/cm²) | ΔTmax Thermocouple à 6s (°C) | $K_i$ (°C/s) |
|---|---|---|---|
| 14 | 380 | 40 | 47.33 |
| 16 | 435 | 50 | 59.16 |
| 18 | 500 | 60 | 80.00 |
| 20 | 576 | 70 | 82.83 |
| 22 | 641 | 80 | 94.66 |
| 24 | 728 | 90 | 106.50 |
| 26 | 804 | 100 | 118.33 |

Le principe de fonctionnement de cet abaque est le suivant. Lorsqu'un utilisateur souhaite calibrer une sonde pour un traitement particulier, il détermine une température souhaitée et une durée d'exposition souhaitée garantissant l'efficacité du traitement du tissu.

Il estime ensuite une température de référence dans le gel et une durée d'exposition de référence dans le gel à partir des températures et durées souhaitées (par exemple en appliquant une fonction de transfert aux températures et durée souhaitées).

En reportant la température de référence estimée et la durée d'exposition de référence estimée sur l'abaque de la figure 5, il en déduit une température de consigne utilisable dans le procédé d'ajustement décrit au point 2.

Bien entendu l'homme du métier saura reproduire le même test avec un gel différent en reprenant la méthode décrite précédemment.

Le lecteur aura compris que de nombreuses modifications peuvent être apportées à l'invention décrite précédemment sans sortir matériellement des nouveaux enseignements et des avantages décrits ici.

Par exemple, dans les modes de réalisation décrit précédemment, le câble électrique destiné à être connecté à une source d'alimentation électrique pour permettre l'alimentation en énergie des transducteurs est intégré à la sonde. En variante, ce câble électrique peut être intégré à la source d'alimentation, la sonde comprenant un raccord électrique de connexion pour permettre son raccordement au câble électrique.

De même, dans la description qui précède, le gel utilisé était un gel thermosensible à base de protéines. Bien entendu, le matériau thermosensible utilisé peut être tout autre gel ou matériau synthétique dont le comportement est représentatif d'un tissu lorsqu'il est soumis à un rayonnement pour augmenter sa température.

Par ailleurs dans la description qui précède, l'étape d'ajustement de la fréquence d'excitation du transducteur était réalisée en obtenant des profils de température du transducteur pour différents signaux d'entrée. En variante, l'étape d'ajustement pourrait être réalisée en obtenant des profils de pression pour différents signaux d'entrée utilisés pour alimenter le transducteur.

**Revendications**

1. Procédé de calibration d'une sonde de traitement comportant au moins un transducteur (21) destiné à générer des ultrasons focalisés de haute intensité dans une zone cible de focalisation, la sonde étant destinée à être connectée électriquement à une source d'alimentation (3) pour la fourniture d'un signal électrique d'alimentation du transducteur, *caractérisé en ce que* le procédé comprend une étape d'ajustement (20) d'une fréquence du signal électrique d'alimentation du transducteur (21) de sorte que la variation entre l'intensité maximale de vibration et l'intensité minimale de vibration du transducteur le long de sa largeur (L) soit minimale, l'étape d'ajustement de la fréquence d'excitation du transducteur comprenant les sous-étapes consistant à :

 o alimenter le transducteur avec une pluralité de signaux d'entrée ayant des amplitudes égales et des fréquences respectives différentes,
 o mesurer des températures ou des pressions en différents points de la zone cible pour chaque signal d'entrée,
 o obtenir un profil de température ou de pression du transducteur pour chaque signal d'entrée,

o sélectionner parmi la pluralité de profils de température ou de pression du transducteur le profil de température ou de pression pour lequel la variation entre la température, respectivement la pression, maximale et minimale du profil de température ou de pression est la plus faible le long de la largeur du transducteur,

o calculer la fréquence du signal électrique d'alimentation du transducteur à partir de la fréquence du signal d'entrée associé au profil de température ou de pression sélectionné.

2. Procédé de calibration selon la revendication 1, lequel comprend en outre une étape de détermination d'une fréquence de résonance du transducteur.

3. Procédé de calibration selon la revendication 2, dans lequel les fréquences de la pluralité de signaux d'entrée sont comprises entre 90% et 110% de la fréquence de résonance du transducteur.

4. Procédé de calibration selon l'une quelconque des revendications 1 à 3, lequel comprend en outre une étape d'ajustement d'une tension du signal électrique d'alimentation du transducteur en fonction d'une température de consigne dans la zone cible de focalisation.

5. Procédé de calibration selon la revendication 4, dans lequel l'étape d'ajustement d'une tension d'excitation comprend les sous étapes consistant à :

   a) alimenter le transducteur avec un signal d'entrée de fréquence fixe, notamment égale à la fréquence d'excitation,
   b) mesurer des températures en différents points de la zone cible de focalisation,
   c) calculer une température moyenne à partir des températures mesurées,
   d) comparer la température moyenne calculée à la température de consigne,
   e) si la température moyenne est supérieure à la température de consigne, alors diminuer l'amplitude du signal d'entrée et répéter les opérations b) à e)
   f) si la température moyenne est inférieure à la température de consigne, alors augmenter l'amplitude du signal d'entrée et répéter les opérations b) à f),
   g) si la température moyenne est égale à la température de consigne, choisir comme tension d'excitation la valeur de la tension du signal d'entrée.

6. Sonde de traitement d'une pathologie comportant des moyens de générations d'ultrasons (2) incluant au moins un transducteur (21) pour générer des ultrasons focalisés de haute intensité dans une zone cible de focalisation, la sonde étant destinée à être connectée électriquement à une source d'alimentation (3) pour la fourniture d'un signal électrique d'alimentation du transducteur,
   *caractérisée en ce que* la sonde comprend des moyens d'ajustement adaptés pour ajuster la fréquence du signal électrique d'alimentation du transducteur de sorte que la variation entre l'intensité maximale de vibration et l'intensité minimale de vibration du transducteur le long de sa largeur soit minimale, lesdits moyens d'ajustement étant configurés pour :

   ○ alimenter le transducteur avec une pluralité de signaux d'entrée ayant des amplitudes égales et des fréquences respectives différentes,
   ○ mesurer des températures en différents points de la zone cible pour chaque signal d'entrée,
   ○ obtenir un profil de température du transducteur pour chaque signal d'entrée,
   ○ sélectionner parmi la pluralité de profils de température du transducteur, le profil de température pour lequel la variation entre la température maximale et minimale du profil de température est la plus faible le long de la largeur du transducteur,
   ○ calculer la fréquence du signal électrique d'alimentation du transducteur à partir de la fréquence du signal d'entrée associé au profil de température sélectionné.

7. Sonde selon la revendication 6, dans lequel les moyens d'ajustement comprennent une carte électronique de pilotage de la source d'alimentation, ladite carte incluant une mémoire contenant au moins un paramètre de calibration obtenu en mettant en oeuvre le procédé de calibration selon l'une quelconque des revendications 1 à 5.

8. Sonde selon la revendication 7, dans laquelle la carte électronique de pilotage est intégrée dans les moyens de génération d'ultrasons.

**Patentansprüche**

1. Verfahren zum Kalibrieren einer Behandlungssonde, die mindestens einen Wandler (21) aufweist, der bestimmt ist, Ultraschall zu erzeugen, der mit hoher Intensität in einen Fokussierungszielbereich fokussiert wird, wobei die Sonde bestimmt ist, für die Bereitstellung eines elektrischen Versorgungssignals des Wandlers mit einer Versorgungsquelle (3) elektrisch verbunden zu sein,
**dadurch gekennzeichnet, dass** das Verfahren einen Schritt des Einstellens (20) einer Frequenz des elektrischen Versorgungssignals des Wandlers (21) umfasst, so dass die Schwankung zwischen der maximalen Vibrationsintensität und der minimalen Vibrationsintensität des Wandlers entlang seiner Breite (L) minimal ist, wobei der Schritt des Einstellens der Erregungsfrequenz des Wandlers die Unterschritte umfasst, die darin bestehen:

   o Versorgen des Wandlers mit einer Vielzahl von Eingangssignalen mit gleichen Amplituden und jeweiligen unterschiedlichen Frequenzen,
   o Messen der Temperaturen oder der Drücke an verschiedenen Punkten des Zielbereichs für jedes Eingangssignal,
   o Erhalten eines Temperatur- oder Druckprofils des Wandlers für jedes Eingangssignal,
   o Auswählen aus der Vielzahl von Temperatur- oder Druckprofilen des Wandlers das Temperatur- oder Druckprofil, für das die Schwankung zwischen der maximalen und minimalen Temperatur beziehungsweise dem Druck des Temperatur- oder Druckprofils entlang der Breite des Wandlers die kleinste ist,
   o Berechnen der Frequenz des elektrischen Versorgungssignals des Wandlers ausgehend von der Frequenz des Eingangssignals, das dem ausgewählten Temperatur- oder Druckprofil zugeordnet ist.

2. Kalibrierungsverfahren nach Anspruch 1, das ferner einen Schritt des Bestimmens einer Resonanzfrequenz des Wandlers umfasst.

3. Kalibrierungsverfahren nach Anspruch 2, wobei die Frequenzen der Vielzahl von Eingangssignalen zwischen 90 % und 110 % der Resonanzfrequenz des Wandlers liegen.

4. Kalibrierungsverfahren nach einem der Ansprüche 1 bis 3, das ferner einen Schritt des Einstellens einer Spannung des elektrischen Versorgungssignals des Wandlers in Abhängigkeit von einer Solltemperatur im Fokussierungszielbereich umfasst.

5. Kalibrierungsverfahren nach Anspruch 4, wobei der Schritt des Einstellens einer Erregungsspannung die Unterschritte umfasst, die darin bestehen:

   a) Versorgen des Wandlers mit einem Eingangssignal mit fester Frequenz, die insbesondere gleich der Erregungsfrequenz ist,
   b) Messen der Temperaturen an verschiedenen Punkten des Fokussierungszielbereichs,
   c) Berechnen einer mittleren Temperatur ausgehend von den gemessenen Temperaturen,
   d) Vergleichen der berechneten mittleren Temperatur mit der Solltemperatur,
   e) wenn die mittlere Temperatur höher als die Solltemperatur ist, Verringern der Amplitude des Eingangssignals und Wiederholen der Vorgänge b) bis e),
   f) wenn die mittlere Temperatur niedriger als die Solltemperatur ist, Erhöhen der Amplitude des Eingangssignals und Wiederholen der Vorgänge b) bis f),
   g) wenn die mittlere Temperatur gleich der Solltemperatur ist, Wählen als Erregungsspannung den Wert der Spannung des Eingangssignals.

6. Sonde zur Behandlung einer Pathologie, die Mittel zur Erzeugung von Ultraschall (2) einschließlich mindestens einen Wandler (21) zum Erzeugen des mit hoher Intensität in einen Fokussierungszielbereich fokussierten Ultraschalls aufweist, wobei die Sonde bestimmt ist, für die Bereitstellung eines elektrischen Versorgungssignals des Wandlers mit einer Versorgungsquelle (3) elektrisch verbunden zu sein,
**dadurch gekennzeichnet, dass** die Sonde Einstellmittel umfasst, die zum Einstellen der Frequenz des elektrischen Versorgungssignals des Wandlers geeignet sind, so dass die Schwankung zwischen der maximalen Vibrationsintensität und der minimalen Vibrationsintensität des Wandlers entlang seiner Breite minimal ist, wobei die Einstellmittel ausgelegt sind, um:

   o den Wandler mit einer Vielzahl von Eingangssignalen mit gleichen Amplituden und jeweiligen unterschiedlichen Frequenzen zu versorgen,

o Temperaturen an verschiedenen Punkten des Zielbereichs für jedes Eingangssignal zu messen,

o ein Temperaturprofil des Wandlers für jedes Eingangssignal zu erhalten,

o aus der Vielzahl vom Temperaturprofilen des Wandlers das Temperaturprofil auszuwählen, für das die Schwankung zwischen der maximalen und minimalen Temperatur des Temperaturprofils entlang der Breite des Wandlers die kleinste ist,

o die Frequenz des elektrischen Versorgungssignals des Wandlers ausgehend von der Frequenz des Eingangssignals, das dem ausgewählten Temperaturprofil zugeordnet ist, zu berechnen.

7. Sonde nach Anspruch 6, wobei die Einstellmittel eine elektronische Steuerkarte der Versorgungsquelle umfassen, wobei die Karte einen Speicher umfasst, der mindestens einen Kalibrierungsparameter enthält, der durch Durchführung des Kalibrierungsverfahrens nach einem der Ansprüche 1 bis 5 erhalten wird.

8. Sonde nach Anspruch 7, wobei die elektronische Steuerkarte in die Ultraschallerzeugungsmittel integriert ist.

**Claims**

1. A method for calibrating a treatment probe including at least one transducer (21) for generating high-intensity focused ultrasound in a target focusing area, the probe being intended to be electrically connected to a power supply source (3) for providing an electric signal for the power supply of the transducer,
**characterized in that** the method comprises a step for adjusting (20) a frequency of the electric signal for the power supply of the transducer (21) so that the variation between the maximum vibration intensity and the minimum vibration intensity of the transducer along its width (L) is minimal, the step for adjusting the excitation frequency of the transducer comprising the sub-steps of:

o supplying the transducer with a plurality of input signals having equal amplitudes and different respective frequencies,

o measuring temperatures or pressures in different points of the target area for each input signal,

o obtaining a temperature or pressure profile of the transducer for each input signal,

o selecting from among the plurality of temperature or pressure profiles of the transducer, the temperature or pressure profile for which the variation between the maximum and the minimum temperature, respectively pressure, of the temperature or pressure profile is the smallest along the width of the transducer,

o calculating the frequency of the electric signal for the power supply of the transducer from the frequency of the input signal associated with the selected temperature or pressure profile.

2. The calibration method according to claim 1, which further comprises a step for determining a resonance frequency of the transducer.

3. The calibration method according to claim 2, wherein the frequencies of the plurality of input signals are comprised between 90% and 110% of the resonance frequency of the transducer.

4. The calibration method according to any one of claims 1 to 3, which further comprises a step for adjusting a voltage of the electric signal for the power supply of the transducer according to a set temperature in the target focusing area.

5. The calibration method according to claim 4, wherein the step for adjusting an excitation voltage comprises the substeps of:

a) supplying the transducer with an input signal with a fixed frequency, notably equal to the excitation frequency,

b) measuring temperatures in different points of the target focusing area,

c) calculating an average temperature from the measured temperatures,

d) comparing the calculated average temperature with the set temperature,

e) if the average temperature is greater than the set temperature, then reducing the amplitude of the input signal and repeating the operations b) to e),

f) if the average temperature is less than the set temperature, then increasing the amplitude of the input signal and repeating the operations b) to f),

g) if the average temperature is equal to the set temperature, selecting as an excitation voltage the value of the voltage of the input signal.

6. A probe for treating a pathology including means for generating ultrasound (2) including at least one transducer (21) for generating high-intensity focused ultrasound in a target focusing area, the probe being intended to be electrically connected to a power supply source (3) for providing an electric signal for the power supply of the transducer, **characterized in that** the probe comprises adjustment means adapted for adjusting the frequency of the electric signal for the power supply of the transducer so that the variation between the maximum vibration intensity and the minimum vibration intensity of the transducer along its width is minimal, said adjustment means being configured to:

   o supply the transducer with a plurality of input signals having equal amplitudes and different respective frequencies,
   o measure temperatures in different points of the target area for each input signal,
   o obtain a temperature profile of the transducer for each input signal,
   o select from among the plurality of temperature profiles of the transducer, the temperature profile for which the variation between the maximum and the minimum temperature of the temperature profile is the smallest along the width of the transducer.
   ◦ calculate the frequency of the electric signal for the power supply of the transducer from the frequency of the input signal associated with the selected temperature profile.

7. The probe according to claim 6, wherein the adjustment means comprise an electronic card for controlling the power supply source, said card including a memory containing at least one calibration parameter obtained by applying the calibration method according to any one of claims 1 to 5.

8. The probe according to claim 7, wherein the electronic control card is integrated into the means for generating ultrasound.

**FIG. 1**

**FIG.2**

**FIG.3**

| $F_{nom}$-200 kHz | $F_{nom}$-100 kHz | $F_{nom}$=21MHz | $F_{nom}$+100 kHz | $F_{nom}$+200 kHz |

**FIG.4**

FIG.5

## RÉFÉRENCES CITÉES DANS LA DESCRIPTION

**Documents brevets cités dans la description**

- WO 2009103721 A

- US 2014024975 A

**Littérature non-brevet citée dans la description**

- **LAFON C ; ZDERIC V ; NOBLE ML ; YUEN JC ; KACZKOWSKI PJ ; SAPOZHNIKOV OA ; CHAVRIER F ; CRUM LA ; VAEZY S.** Gel phantom for use in high-intensity focused ultrasound dosimetry. *Ultrasound Med Biol.,* 31 Octobre 2006, vol. 31 (10), 1383-1389